# EUROPEAN PATENT SPECIFICATION

(11) **EP 1 635 883 B1**
(45) Date of publication and mention of the grant of the patent: **03.01.2007**
(21) Application number: 04739939.9
(22) Date of filing: 16.06.2004
(51) Int. Cl.: A61L 11/00, A61L 2/12, B65B 7/00

(54) **MACHINE AND METHOD FOR TREATING PRODUCTS WITH MICROWAVES**
MASCHINE UND VERFAHREN ZUR BEHANDLUNG VON PRODUKTEN MIT MIKROWELLEN
MACHINE ET PROCEDE POUR TRAITER DES PRODUITS AVEC DES MICRO-ONDES

(30) Priority: 30.06.2003 IT BO20030402
(43) Date of publication of application: 22.03.2006
(73) Proprietor: Salda, Luciano, 41058 Vignola (MO) (IT)
(72) Inventor: Salda, Luciano, 41058 Vignola (MO) (IT)
(74) Representative: Porsia, Dino
(86) International application number: PCT/EP2004/006472
(87) International publication number: WO 2005/002639

(56) References cited:
- EP-A- 0 410 306
- WO-A-91/15247
- WO-A-03/049864
- PATENT ABSTRACTS OF JAPAN vol. 2003, no. 10, 8 October 2003 (2003-10-08) & JP 2003 175094 A (EBARA CORP), 24 June 2003 (2003-06-24)

## Description

The systems used at present for the microwave treatment of products in general, and of waste in particular, are usually of the continuously operating type and require continuous supply of the said waste to a treatment chamber where the said waste can be kept temporarily or can be moved by the action of conveyor means, and where they are heated by the action of the microwaves to the desired temperature and for the desired time, and are then discharged continuously to means of disposal. These system are expensive and difficult to operate, and are unsuitable for cyclical treatment of the variable quantities of solid waste produced, for example, in a hospital environment, which at present are collected in suitable cardboard or corrugated plastic boxes and which are intended for disposal in authorized incinerators. There are also intermittently operating microwave treatment systems, typically used in laboratories, which are not organized on the basis of automatic supply and discharge of waste to and from the treatment chamber.

EP-A-0 410 306 discloses an apparatus and a method for the treatment of hospital waste wherein a sealed container comprising the waste is conveyed into a sterilisation chamber where the waste is subjected to microwave radiation and thereafter removed from the sterilisation chamber.

In relation to the specific problem of sterilizing hospital waste or similar problems, in order to ensure that the said waste can be neutralized in a limited time after its formation, with advantages in the containment of the propagation of any bacterial load of the waste, and to ensure that after treatment the said waste can be disposed of at low cost in the same way as ordinary waste, the invention proposes an easily controlled cyclically operating machine using highly reliable technology, based substantially on the following proposed solution. The machine comprises a plurality of identical containers, of suitable shape and capacity, preferably with flared mouths and round cross sections, which, by the action of a carousel or any other suitable conveyor means, are cyclically made to interact with the following operating stations: a) a first station which shreds the boxes with the waste, to create a finely shredded product which, by suitable means, is fed into each container in predetermined, constant amounts, is compacted, and is suitably moistened, the mouth of the container being protected and kept clean by suitable means; b) a second station in which the mouth of the container is closed to form a seal by a piston which communicates by means of a wave guide with the magnetron which generates the microwaves required for the sterilization of the waste in the container. Suitable means control the pressure and if necessary the temperature within the container, in such a way that the waste is treated at the specified temperature and for the time required to neutralize any bacterial load present in the waste, while on completion of sterilization the treatment container is brought to atmospheric pressure and removed from the said piston; c) a station, if required, for removing any excess liquid contained in the sterilized waste, while the mouth of the container is suitably protected and kept clean; and d) a station which discharges the treated waste from the container, while the mouth of the said treatment container is protected and kept clean by suitable means, so that it can subsequently interact and form a seal with the piston of the sterilization station.

Further characteristics of the present invention, and the advantages derived therefrom, will be made clearer by the following description of a preferred embodiment of the invention, illustrated purely by way of example and without restrictive intent in the figures on the attached sheets of drawing, in which:
- Figs. 1 and 2 show in longitudinal section, with parts in view and with various details of construction, one of the treatment containers used by the machine;
- Fig. 3 shows other details of the container of Figure 1, in a section taken through the line III-III;
- Fig. 4 shows in a side view and with parts in section the carousel on which the containers shown in the preceding figures are mounted, and shows in particular the waste sterilization station;
- Figs. 5 and 6 show, in front and side elevation respectively, with parts in section, the initial and final parts of the station for shredding the waste and for feeding them into the treatment containers with appropriate moistening;
- Fig. 7 shows, in a side elevation with parts in section, the station for drying the treated waste;
- Figs. 8 and 9 show, in side elevation and in plan view from above respectively, the station for discharging the waste from the treatment containers and for cleaning the said containers, if necessary, before their return to the operating cycle.

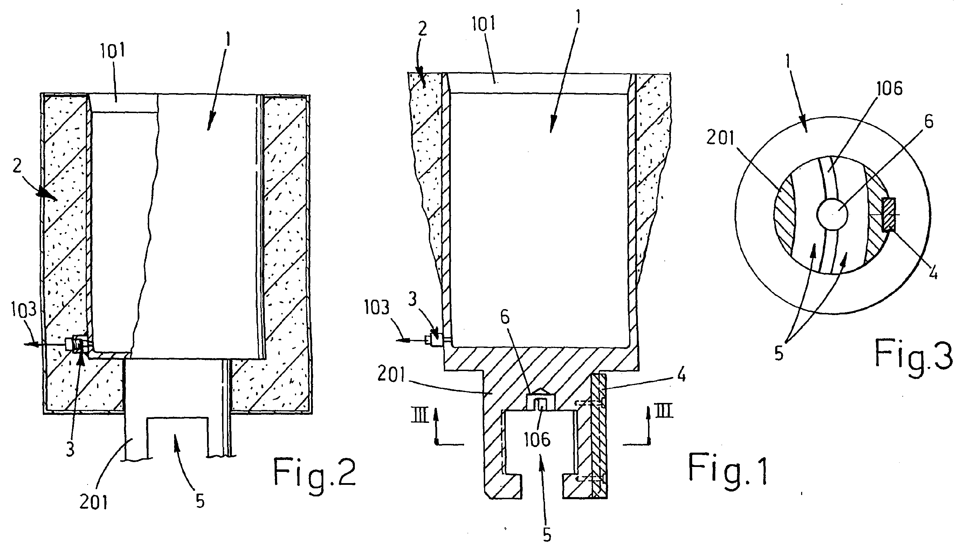

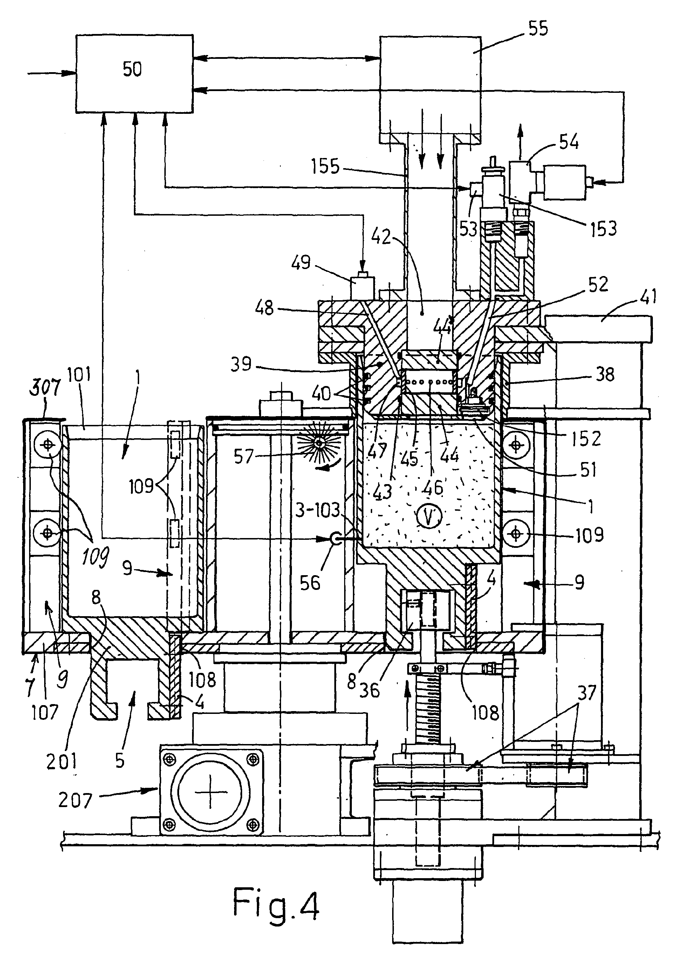

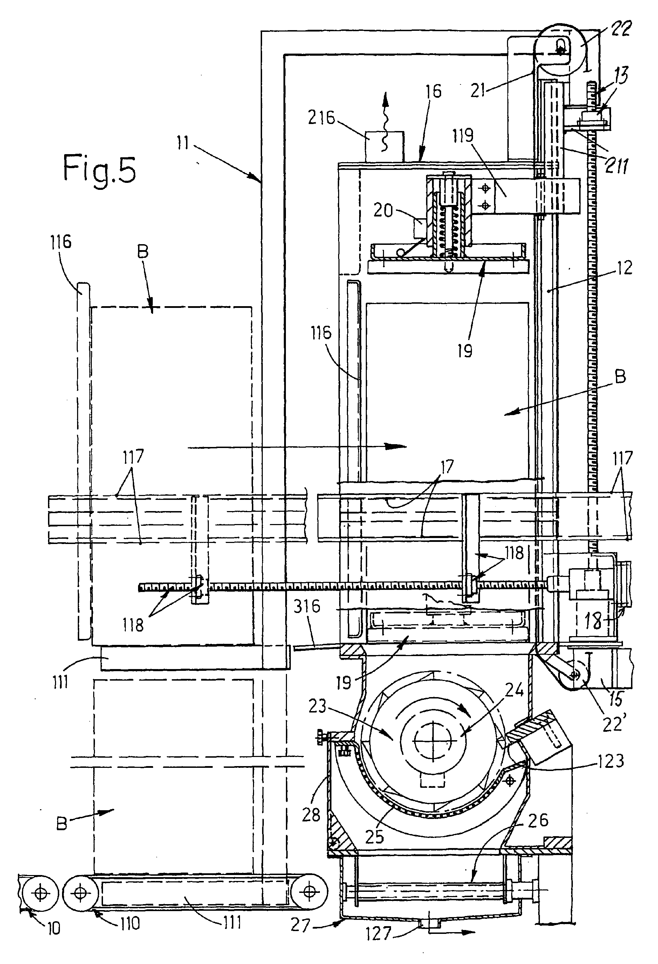

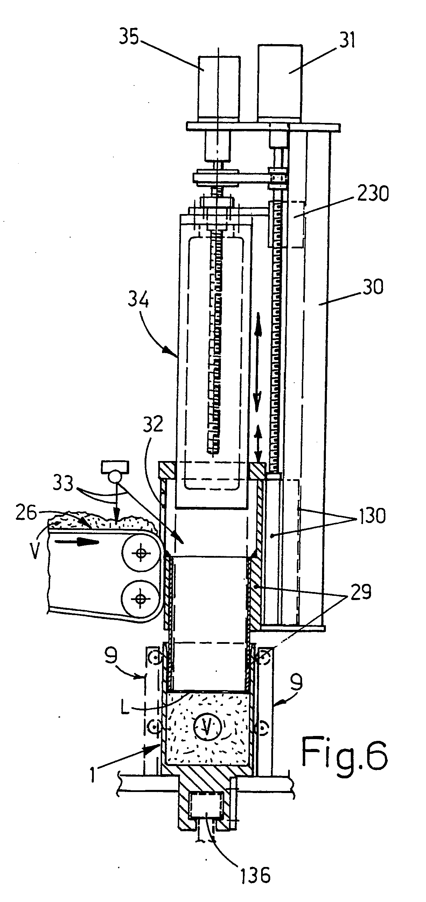

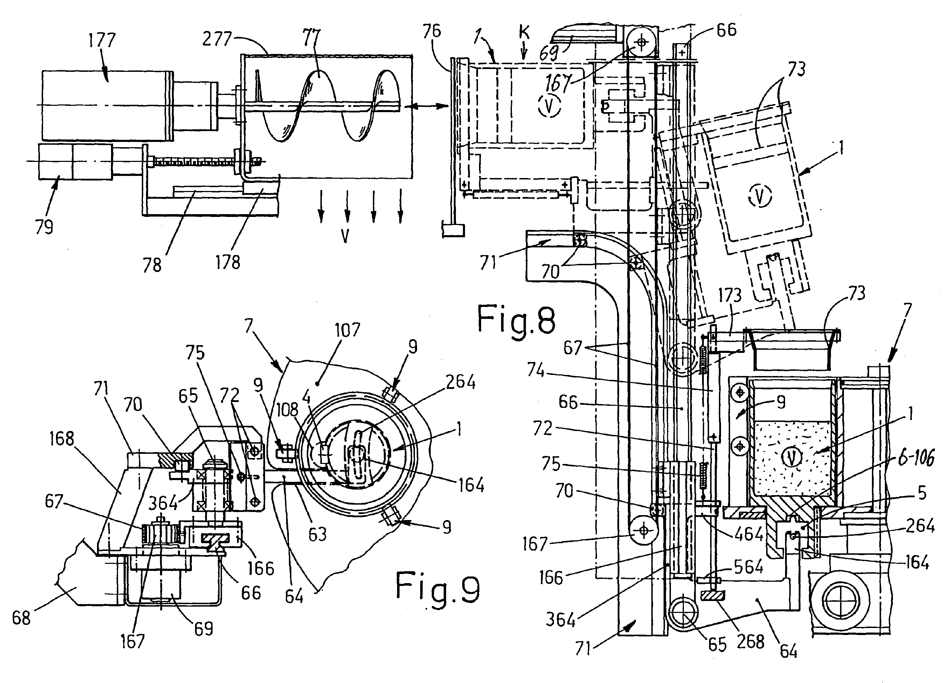

Figure 1 shows how the machine uses at least four or three (see below) cylindrical steel containers 1, with suitable dimensions, for example with an internal diameter of approximately 130 mm and with a height of approximately 190 mm, with suitably rounded lower internal angles and with an internally flared upper edge 101, preferably having an outer jacket 2 for thermal insulation (see also Fig. 2) and having if necessary, at least at the bottom, a housing for a temperature probe 3 which, for example, has a suitable plug 103 projecting from the said jacket 2 (see below). To allow for the type of handling for which they are intended in the present example, each of the containers 1 has an external axial shank 201 on its base with a lateral key 4 and with an underlying median aperture 5 in the form of a C rotated through ninety degrees, with a curve profile in plan view (Fig. 3) and having in the centre of its arched structure a round recessed indentation 6, intersected diametrically by a linear longitudinal median recessed indentation 106. For simplicity of representation, the containers 1 are illustrated without their jackets 2 in the other figures of the drawing. The containers 1 are mounted, for example, vertically and at equal angular intervals, on a carousel 7 with a vertical axis, as shown in Figure 4, which is rotated on command, by means of its intermittent rotation system 207, with an angular amplitude equal to that present between the containers 1 located on it. The carousel 7 comprises a platform 107 with holes 8 suitably shaped for the passage of the shanks 201 and with enlargements 108 (see also Fig. 9) for the passage of the keys 4 of the containers 1, which thus rest with their bases on the said platform 107 and have their apertures 5. in vertical radial planes with respect to the carousel 7 and therefore always orientated correctly with respect to the stationary means which are required to engage with them cyclically and then disengage from them (see below). For this purpose, the curvature in plan view of the aperture 5 has its centre on the axis of the carousel 5. Vertical bars 9, spaced at equal angular intervals, with free-running rollers 109 having horizontal axes, are fixed on the said platform 107, at least three of these bars being provided for each container 1, to form true guide cages for the said containers. The carousel 7 can also be provided with a suitable casing 307 and other means to allow the various components of the carousel to be cleaned conveniently if necessary before each maintenance operation performed on it, for example by the introduction into the said casing of water vapour and/or other products suitable for the purpose, the whole arrangement being evident to persons skilled in the art and suitable for implementation by them in various ways. With reference to Figures 5 and 6, it will be noted that the first station with which the containers 1 are made to interact is that designed for loading the waste to be treated into the said containers. This station can be associated with means which automatically feed the boxes of waste to the shredding device. The boxes B filled with waste are, for example, placed in Indian file on a conveyor 10 which feeds the leading box to a final accelerating conveyor 110 which separates this box from the following one and which positions it correctly on top of the pair of parallel horizontal prongs 111 of an elevator 11. It should be understood that, in reality, the conveyor system 10, 110 can be rotated through ninety degrees from the position shown in Figure 5, still with the final conveyor 110 positioned between the prongs of the elevator 11, but perpendicularly to these. The elevator 11 is associated with a slider 211 which slides on a vertical guide 12 and which is moved, for example, by a male and female thread system 13 driven by a reversible motor 15, assisted by limit microswitches which are not shown. The aforesaid guide 12 is fixed laterally to a parallelepipedal hopper 16 which is rectangular in plan, is large enough to contain at least one box B, and has a lateral hatch 116, whose height is greater than that of a box B, associated with a horizontal assembly consisting of a guide and slider 17, 117, the said slider being connected for example to a male and female thread drive system 118 powered by a reversible electric motor 18 and by means of limit microswitches which are not shown. An arm 119 fixed to the slider 211 enters the hopper 16 through a longitudinal aperture formed in the lateral wall opposite that closed by the hatch 116, and supports in the hopper a sprung horizontal presser 19, with a sensor 20 which senses the load of the springs and which is described more fully below. The ends of a band 21 of steel or other material, which closes the aperture of the hopper through which the said arm passes, and which runs around free-running stationary pulleys 22, 22', are fixed to the arm 119. At the base of the hopper 16 there is provided at least one shredder 123 with fixed blades which interact with the peripheral blades of a shredding cylinder 23 which rotates about its own horizontal axis, usually in the clockwise direction with respect to Figure 5, and which is driven by a reversible electric motor 24 with thermal protection. Under the rotor 23 of the shredder there is located a static or vibrating screening grid 25, which allows only the shredded waste V with the predetermined particle size to pass through, together with any liquid present in the waste. A conveyor means 26 of any type suitable for the purpose, which if necessary can allow any excess liquid to flow into an underlying tank 27 from whose discharge pipe 127 the said liquid is drained and can, for example, be circulated to moisten new material is placed in a parallel manner underneath the grid 25. The conveyor 26 can consist of a belt, perforated or porous if necessary, or a system with one or more vibrating channels in a cascade formation. The base of the hopper 16 is connected by means of a casing 28 to the tank 27, to prevent any dispersion of dust due to the shredding operation. For this purpose, it is possible to provide, in the upper part of the hopper 16 and/or in any other suitable position, ducts 216 connected to suction, filtration and sterilization means, which maintain a precise negative pressure in the whole shredding system. The station described above operates as follows. When the presser 19 is at the lower limit of its travel and at the point of maximum closeness to the shredder 23, the prongs 111 are positioned below and at the sides of the conveyor 110, which is then automatically activated to position a box B containing waste for disposal on top of it and above the said prongs 111. Weighing and monitoring means for other purposes can be provided in the area of the conveyor 110, to check whether or not the content of the box conforms to the safety conditions specified for correct operation of the machine, and, if these conditions are not present, means (not shown) are provided to remove the box from the conveyor 110. The top of the box placed on the conveyor 110 is located at a height such that it does not interfere with the lower part of the hatch 116 which is located above it and which, in the next step, is translated horizontally into the position shown in broken lines, to open the hopper 16 in which the shredder 23 has previously been stopped. In the next step, the slider 119 is raised to lift both the presser 19 and the elevator 11, and the upward travel is stopped when the presser is in the upper part shown in solid lines and the prongs 111 are in a position of substantial alignment with the base of the hopper 16. At this point, the motor 18 is restarted to translate the hatch 116 towards the hopper 16, and in this step the hatch itself acts as a pusher and transfers the box B into the said hopper, making it slide on the prongs 111 and then on a bridge 316 which is, for example, integral with the said hopper. When the box B has been fed in, the shredder 23 starts and the motor 15 is reversed to lower the presser 19 which executes its pushing action on the box B, to keep it progressively in correct interaction with the said shredder which gradually shreds it together with all of its contents. The descent of the presser 19 is stopped automatically when the sensor 20 detects that the springs with which the said presser is provided are stressed, and restarts automatically when the said sensor detects the opposite condition and the travel continues to the lower limit which is detected by a limit microswitch. If the thermal device controlling the motor 24 detects excessive force during the step of shredding the box B and its contents, means are provided to ensure that this motor stops, that the presser 19 is suitably raised and that the said motor then restarts in reverse, in such a way as to free the shredder. After a predetermined time, the shredder starts to rotate again in the clockwise direction and the presser 19 starts to descend again to restart the normal operating cycle. The operation of the shredder is clearly also controlled by means (not shown) which monitor the state of accumulation of the shredded waste on the conveyor 26, for example when this conveyor is stopped if necessary after the step of filling a treatment container 1. Figure 6 shows that, when the carousel 7 positions a container 1 in the filling station, the said container is placed under and in axial alignment with a sleeve 29 mounted on a slider 130 which slides on a vertical fixed guide 30 and which is connected to raising and lowering means consisting, for example, of a male and female thread unit, a reversible motor 31 and limit microswitches (not shown). On command, the sleeve 29 is lowered in such a way that its lower end engages in the mouth of the container 1 to protect it from contamination and to act as a funnel for connection to the means of feeding the waste to be sterilized. This is because the sleeve 29 is provided with a lateral aperture 32, aligned with the conveyor 26, which, when the said sleeve is lowered, is placed under the active branch of the said conveyor, while, when the sleeve is raised, the said aperture is raised and the sleeve also acts as a means of stopping the shredded waste placed on the conveyor 26. When the sleeve 29 is in the low position, the finely shredded waste V is discharged from the conveyor 26 into the container 1. Suitable means, indicated schematically by the arrow 33, are provided to spray a precise quantity of liquid, for example water, on to the waste V which is fed into the container 1 in the filling step. The upper end of the sleeve 29 is normally engaged by the lower end of a cylindrical piston 34, whose diameter is slightly less than the internal diameter of the container 1, and whose upper end is connected to a slider 230 which slides, for example, on the guide 30 and which is connected to means of raising and lowering, of the male and female thread type for example and with a reversible electric motor 35 with electronic speed, phase and torque control, for example a brushless motor. In a cyclical way, the conveyor 26 is stopped and the piston 34 is lowered to compress the waste in the container 1, torque control being provided at least in the final stage, in such a way as to form a thoroughly compacted layer of the said waste in the treatment container, for example at a constant level indicated by L, by means of any suitable operating logic which can be easily implemented and determined experimentally. It is important that the waste V introduced into the container 1 should be sufficiently moist, and it is therefore necessary to take into account the capacity and activation time of the feed means 33; it is possible to act on the waste while it is still located on the conveyor 26, on the assumption that excess liquid will run out of the said waste by gravity, or the liquid level within the container 1 can be monitored and/or adjusted, for example by means located in the piston 34. For this purpose, it should be noted that in the final stage of compression of the waste in the container 1, the waste acts like a sponge which is squeezed by the piston 34, as a result of which the liquid contained in the said waste tends to rise to the surface. By using sensors (not shown) fitted in the piston 34, it will be possible to detect the quantity of liquid present in the pressed waste and by using special means in the supply system 33 it will be possible to regulate this quantity of liquid, the whole arrangement being evident to persons skilled in the art and easily applicable by them. The waste introduced into the container 1. can be moistened in a different way from that illustrated, by using means located in the piston 34 which supply the liquid to the waste during the active strokes of the said piston which compress the said waste in the treatment container, instead of by using the aforesaid supply systems 33. When the container 1 has been filled, and after the piston 34 has returned to its upper rest position, the sleeve 29 is also raised, removed from the container 1 and returned to the start of cycle position. The weight of the container 1 when filled with waste is such that the container is kept in the low position by gravity, even if there is a slight degree of friction between the sleeve 29 and the mouth of the said container. If necessary, however, suitable means shown in broken lines and indicated by the number 136 can be provided on the base of the carousel to engage the lower aperture 5 of the shank of the container 1, to prevent the container 1 from following the sleeve 29 in the raising step and also to oppose the thrust exerted by the piston 34 in the step of compressing the waste, in order to avoid the anomalous discharge of this thrust on to the rotating platform 107 of the carousel 7.

With reference to Figure 4, it will be seen that, while the carousel 7 positions a new container 1 in the aforesaid filling station, the container 1, which is leaving this station and is filled with the compacted mass of waste V, is transferred to the sterilization station which comprises, in particular, a mushroom-headed device 36 whose head engages in the lower aperture 5 of the shank of the container 1 and which, under the action of axial means of movement, for example means of the male and female thread type with a reversible motor 37, is raised through a predetermined necessary distance to push the upper part of the said container 1 into a protective and shielding sleeve 38 which surrounds, with a degree of clearance, a cylindrical piston 39 which enters, with a seal formed by its lateral gaskets 40, into the mouth of the said container 1 until it is close to the level L of the mass of waste V to be treated, this assembly 38, 39 being integral with a fixed support structure 41 which is fastened, for example, to the base of the carousel. The piston 39 has an axial cavity 42 of suitable shape and size, which terminates below in an enlarged portion in which are housed, with the interposition of corresponding lateral sealing gaskets 43, a pair of diaphragms 44, 44' made of quartz or other suitable material which is permeable by microwaves but not by fluids, kept at the requisite distance from each by an annular spacer 45 having radial through holes 46 which communicate with an outer annular recess 47 to which is connected an ascending channel 48 formed in the body of the piston and connected to a pressure transducer 49 which transmits the electrical signal corresponding to its reading to a processor 50 which controls the operation of the machine or of the unit in question. The quartz pieces 44, 44' are, for example, secured in place by a flange 51 fixed on the lower flange of the piston 39, but it should be understood that other suitable means can be used for this purpose. A pressure outlet 152 opens on the lower face of the piston 39 and is connected to an ascending channel 52 which branches into at least two passages, one of which is connected to a maximum pressure valve 153 and in parallel to a pressure transducer 53 which transmits the corresponding electrical signal to the processor 50. The other passage of the channel 52 leads to a solenoid valve 54 which is controlled by the said processor 50 and which discharges into a protected area, for example into the container (not shown) from which water is drawn for moistening the waste V during the filling of the containers 1. The axial cavity 42 of the piston 39 is connected by means of a waveguide 155 to the generator 55 which produces the microwaves required for the sterilization step, for example a conventional cavity magnetron of suitable power which operates in the frequency band around 2.45 GHz. To optimize the operation of the generator 55, the generator can if necessary be provided with known means which read the amount of waves reflected towards the said generator. The operation of the generator 55 is also controlled by the processor 50 which, through a circuit connected to a socket 56 which can be provided for connection to the plug 103 of the container 1, if present, can also receive the electrical signal relating to the temperature at the base of the said container 1 inserted into the sterilization station. The presence of the temperature probe 3, 103 is optional. It was provided in the prototype of the machine in question, in order to monitor in a safe way the operation of the said machine and to optimize the sterilization cycle, since the graph of sterilization as a function of time is not linear. In the production of the machine on an industrial scale, the said temperature probe can be omitted, since this would yield evident advantages in the construction and handling of the containers 1, and since the temperatures within the said containers can be deduced from the parameter of pressure which is directly proportional to the temperature and correlated with it. As the temperature rises, the liquid contained in the waste evaporates and the pressure increases. The station shown in Figure 4 operates in the following way. When the container 1 has been coupled to the piston 39, the processor 50 causes the source 55 to be activated. For the sterilization step, a predetermined temperature must be reached within the container 1 and this temperature must be maintained for a predetermined time. For example, in a moisture-saturated environment, a temperature of approximately 150°C maintained for approximately 9-10 seconds causes the assured destruction of any bacterial load present in the mass of waste in the container 1, without any combustion phenomena occurring in this mass. In the absence of the temperature control 56, 103, 3, when the processor 50 detects, through the pressure sensor 53, the attainment of a specified pressure level which significantly corresponds to the attainment of the said temperature, for example a pressure of approximately 8 bars, the source 55 is kept active for the aforesaid period of maintenance, which allows the temperature to be spread uniformly throughout the container, plus the sterilization time for the specific temperature, while the pressure within the container 1 is maintained around the said specified level by the modulated opening of the solenoid valve 54 which discharges water vapour. However, in the presence of the temperature control 56, 103, 3, which is located in the most critical part of the container 1, when the processor 50 detects the attainment of the specified temperature in the container, the source 55 is kept active for the time required for sterilization in the most critical part monitored by the temperature control system, while the pressure within the container 1 is maintained around a specified level, approximately 8 bars for example, by the modulated opening of the solenoid valve 54 which discharges water vapour. At the end of the sterilization cycle, the source 55 is switched off, the solenoid valve 54 is opened to discharge the residual pressure, and finally the container 1 is lowered and removed from the piston 39. During the active stage of operation of the sterilization station, if the pressure in the container 1 exceeds predetermined safety levels, the maximum pressure valve 153 is triggered. On the other hand, if the sensor 49 detects a pressure level above zero, the sterilization unit is depressurized and opened, and an alarm signal is generated to indicate the occurrence of the problem which is probably caused by the breaking of the seal 43 of the lower quartz piece 44 or by the breaking of the quartz piece itself. When the container 1 has been lowered, the carousel 7 rotates through ninety degrees, and a brush, or other suitable means 57 mounted on the said carousel, passes under the lower quartz piece 44 of the sterilization piston 39 to clean it. It should be understood that, in order to limit the reflection of the microwaves towards the generator 55, the latter can be positioned in any suitable way which may differ from what is illustrated, and that the bases of the containers 1 and/or the lower surface of the piston 39 can be shaped in a form which is not flat and which is suitable for the purpose, for example in a curved form, the whole arrangement being evident to persons skilled in the art and easily applied by them.

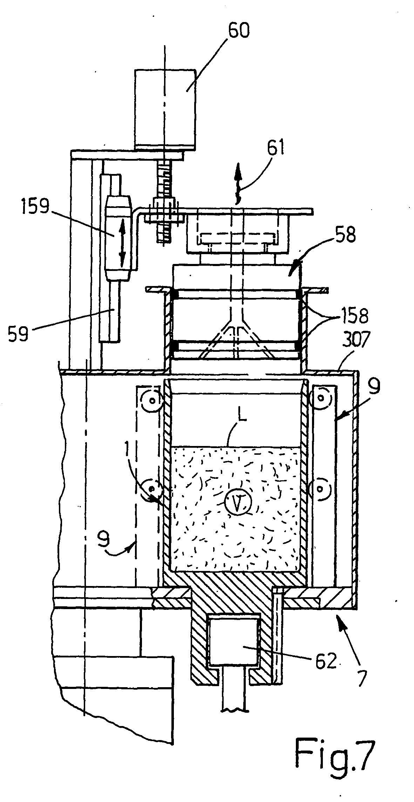

The operating station following the sterilization station can comprise means for removing the water from the sterilized waste V, this water being possibly recovered for the steps of moistening future waste to be treated. These means are shown in Figure 7, and comprise a piston 58 with sealing gaskets 158, mounted on a slider 159 which slides on a vertical fixed guide 59 and which can be raised and lowered by means of a male and female thread system and a reversible electric motor 60, this motor being of the type with electronic speed and phase control if required, in such a way that it can be operated with appropriate acceleration and deceleration gradients. The piston 58 is gas-permeable and is connected to a vacuum pump represented schematically by the arrow 61 and to valve means (not shown), by means of which, when the piston 58 is inserted into the mouth of the container 1, the liquid contained in the waste can be drained off and the said container can then be returned to atmospheric pressure before the step of extraction and removal of the said piston 58 to the rest position. The aperture 5 of the shank 201 of the container can advantageously be engaged by the head of a mushroom-headed device 62 mounted on the base of the carousel, to keep the said container axially stationary in opposition to the axial movement which may be caused by the steps of insertion and extraction of the piston 58 and by the cavitation phenomena created by the vacuum pump 61. To improve the dehydration action provided by the vacuum pump and to ensure efficient cooling of the treated waste, each treatment container can be provided in its base with at least one one-way valve (not shown), which opens automatically in this step only, the whole arrangement being evident to persons skilled in the art and easily applicable by them. The station of Figure 7 is followed by the station for discharging the sterilized waste V from the container 1, as will now be described with reference to Figures 8 and 9. Figure 9 shows that the platform 107 of the carousel 7 has slots 63 opening on its periphery and having their other ends opening tangentially into the seats 8 for housing the shanks 201 of the containers 1. When a container 1 reaches this station, the aperture 5 of its shank 201 is entered by a vertical pin 164 which carries a curved cross-piece 264 running across its end, these components being aligned with the recesses 6 and 106 respectively on the top of the said aperture 5 (Fig. 3). The said pin 164 is integral with a small arm 64 aligned vertically with the said slot 63 and pivoted transversely at 65 on a carriage 166 which slides on a vertical fixed guide 66 and which is associated with a raising and lowering means, for example the vertical branch of a toothed belt conveyor 67 running around the pulleys 167 supported rotatably by the structure 68 which also supports the said guide 66, one of these pulleys being connected to and driven by a reversible electric motor 69, preferably of the type with electronic speed and phase control. When viewed from the side as in Figure 8, the arm 64 is L-shaped, with one portion 364 orientated upwards and having fixed to its upper end a cross-piece 464 which is T-shaped in plan view, whose foot carries a rotatable roller 70 with a horizontal axis, which runs in a rectilinear fixed guide 71 having an appendage 168 fixed to the frame 68. The guide 70 has a large lower vertical rectilinear portion, followed by a portion curving away from the carousel and a short final portion which is rectilinear and practically horizontal. The end of the cross-piece 464 carries guide seats on its enlarged part above the arm 64, and corresponding guide seats are provided in a gusset 564 fixed to the said arm, and a pair of vertical bars 72 slide in these seats, the upper ends of these bars being fixed to the appendage 173 of a small hopper 73 which is held at a small distance from the container 1 to be emptied, for example by the interaction of the lower end of at least one of the said bars 72 with a fixed appendage 268, or by another suitable method. The hopper 73 is provided with a cylindrical lower part, designed for insertion with a good seal into the mouth of the container 1 to be emptied, and its upper end terminates in a conical and outward-diverging part. Tubes 74 of adequate height, acting as spacers in the way described below, are mounted on the portions of the bars 72 lying between the appendage 173 and the cross-piece 464. The number 75 indicates at least one cylindrical helical spring having one end fixed to the said appendage 173 and the other end fixed to the cross-piece 464, in such a way as to keep the hopper 73 pushed towards the arm 64. When a container 1 reaches the discharge station, the processor of the machine causes the motor 69 to be started in order to raise the carriage 166. The arm 64 rises and, with the pin 164 and the key 264, engages with the corresponding recessed parts of the base of the container 1 and raises the container which slides on its external guides 9. The hopper 73 remains stationary and is inserted into the upper mouth of the container 1 as the latter is raised. When the cross-piece 464 touches the lower end of the tubes 74, the hopper 73 has completed its travel for insertion into the container 1 and follows the latter in the raising step. Being held securely by the hopper 73 and by the lower unit 164, 264, the container 1 leaves the corresponding guides 9 of the carousel, and in the next step the roller 70 starts to interact with the curved part of the guide 71, as a result of which any further raising of the slider 166 causes a rotation of the container 1 about the pivot 65, this rotation ending when the said container reaches the horizontal position indicated by K and in broken lines, and when the hopper 73 touches the aperture of a vertical fixed protector 76, associated with means of collecting and evacuating the sterilized waste, these means not being shown, since they are irrelevant to the understanding of the invention. When the container 1 has reached the said horizontal position, it is aligned axially with a screw 77 driven by a motor 177, provided with a fixed and downwardly open casing 277, this assembly being mounted on a slider 178, which slides on a horizontal guide 78 aligned with the axis of the said screw, and which is connected to advancing and withdrawing means consisting, for example, of a male and female thread assembly and by a motor 79, preferably of the type with electronic speed and phase control. At the correct time, the motor 177 and then the motor 79 are activated to rotate the screw 77 and to insert it into the container 1, in order to extract the sterilized waste from the container. The operating cycle of the screw can be that which is most suitable for the purpose. On completion of discharging, it is possible to provide a step of cleaning the container 1 with a rotary brush, with suction means and/or with any other suitable means which are not illustrated. On completion of the discharge of the waste from the container 1, the motor 69 is made to rotate in a direction which is the reserve of the preceding direction, to return the said container to the corresponding guides 9 of the carousel 7 and to make the gripping parts 73, 164, 264 also return to the rest position shown in Figure 8 in solid lines, in such a way that the carousel 7 can rotate through ninety degrees to repeat the cycle. It is to be understood that the construction of the machine as described can be subjected to numerous variations and modifications, relating, for example, to the use of means other than a carousel for transferring the treatment containers to the various operating stations, for example means of the transfer device type. The use of these means could, for example, be useful for simplifying the machine, since the containers would be placed naturally in a horizontal position and even with an inverted orientation while running around the sprockets of the transfer device and during the travel along the lower branch of the said transfer device, where it would be possible to provide, together with means for emptying the containers 1, means for cleaning the said containers and for testing the operation of any temperature probes 3 fitted in them.

## Claims

1. Machine for treating products with microwaves for sterilizing solid hospital waste which is stowed in suitable cardboard or corrugated plastic boxes, comprising a plurality of robust treatment containers (1) of any suitable shape and capacity, open at one end at least and suitably pressure- and heat-resistant, which, by the action of a carousel (7) with a vertical axis or any other suitable conveyor means of the machine, adapted to be cyclically made to interact with at least the following operating stations of the machine: a first station adapted to shred the boxes with the waste, to create a finely shredded product (V) which, by suitable means of the machine, is fed and compacted in predetermined constant amounts, suitably moistened, into each container (1); a second station adapted to temporarily seal the mouth of the container, with the compacted, constant and moistened mass of waste inside, by suitable means (39) which communicate by means of a wave guide (155) with a source (55) adapted to generate the microwaves with the necessary power and for the necessary amount of time to sterilize the said moist mass of waste (V), this station being equipped at least with means for monitoring and controlling the pressure generated in the container during the microwave heating and with means for depressurizing the container at the end of the cycle; and a final station adapted to discharge the sterilized waste from the container.

2. Machine according to Claim 1, in which a third station for dehydrating or drying the sterilized waste to a sufficient degree is provided between the second and final stations.

3. Machine according to Claim 1, in which means for cleaning the treatment containers (1) are provided in the final operating station or after this station.

4. Machine according to Claim 1, in which means are provided for sanitizing the treatment container (1) and if necessary all parts of the machine which operate in contact with the waste to be sterilized, before the said parts are adapted to be subjected to maintenance operations.

5. Machine according to Claim 1, in which the treatment containers (1) are provided with an outer jacket for thermal insulation (2).

6. Machine according to Claim 1, in which each treatment container (1) carries at least one temperature probe (3) with a corresponding external outlet (103) for removable, rapid and temporary connection to means adapted to detect the temperature inside the said container during the step of sterilizing the waste contained in it, this probe being preferably positioned as closely as possible to the base of the said container.

7. Machine according to Claim 1, in which each treatment container (1) consists of a steel cylinder of suitable dimensions, with a round cross section, having a suitable inner flare (101) on its upper mouth and a suitable rounding of the angular region of the connection between the side surface and the base of the said container.

8. Machine according to Claim 1, in which the treatment containers (1) are mounted vertically and with their mouths upwards on a carousel with a vertical axis (7), with the possibility of sliding between vertical guides (9, 109) fixed on this carousel, and each of the said containers is provided externally on its base with an axial shank (201) which has a key (4) passing through the shaped through hole (8, 108) in the platform (107) of the carousel, below which the said shank (201) projects with a lower aperture (5) in the shape of a letter C rotated through ninety degrees, and which is kept by the presence of the said key on a theoretical plane containing the axis of rotation of the carousel, in such a way that this aperture can be engaged and disengaged correctly by the head of opposing and/or moving means in the shape of a mushroom or a T, located in the operating stations of the machine.

9. Machine according to Claim 1, in which the first operating station comprises:
- Means (10-18) for feeding the boxes with the waste (B), one at a time, into the loading hopper (16) of a shredder (23, 123), with a presser (19) adapted to push progressively and in a controlled way the said box towards the shredding means;
- Screening means (25) which allow only the waste shredded to a sufficiently fine and predetermined particle size to pass downwards;
- Conveyor means (26) adapted to collect the shredded waste leaving the said screening means and carry it to a position above the container (1) to be filled;
- Means (27, 127) adapted to collect and drain the excess liquid from the shredded waste;
- Means (33) for correctly moistening the shredded waste to be inserted and/or already inserted into the treatment container (1);
- A vertical sleeve (29) whose lower end is inserted temporarily by any suitable means for a precise distance into the mouth of the treatment container (1) which is stationary in the loading station, and which is provided with a lateral aperture (32) through which passes the shredded waste arriving from the said conveyor means (26);
- A piston (34) mounted on a vertical slider (230) driven, for example, by a motor (35) with electronic speed, torque and phase control, adapted to cyclically make the said piston travel along the said guide and compress the shredded waste placed in the treatment container;
- Means for cyclically loading a quantity of shredded waste into the treatment container (1), with values of height and density which are as far as possible constant and predetermined, and means for ensuring that this load of waste contains a sufficient quantity of water.

10. Machine according to Claim 9, in which suitable means (216) are provided to keep the whole environment in which the waste is shredded at a precise negative pressure with respect to the external environment, and to remove and recover in a safe and hygienic way any dust resulting from the said step of shredding the waste.

11. Machine according to Claim 1, in which the station for sterilizing the load of shredded waste placed in the treatment containers (1) comprises:
Means (36, 37) for temporarily raising the container (1) with the waste to be treated in such way that a fixed piston (39) enters the said container with a lateral seal and for a precise distance, this piston having an axial cavity (42) which is connected by a wave guide (155) to the source (55) adapted to produce the microwaves at the frequency and with the power required for the sterilization step;
- In the lower part of the said axial cavity of the piston (39), at least two diaphragms, (44, 44') of microwave-permeable but not fluid-permeable material, such as quartz, being provided at a precise distance from each other, and the chamber formed between these two diaphragms, delimited by a suitable spacer (45), being designed for connection through suitable passages (46-48) to a pressure sensor (49) connected to the processor (50) adapted to control the operation of the system;
- On the lower face of the said piston (39) opening a pressure outlet (152) which is connected, by means of a channel (52) having at least two branches, to a safety valve or maximum pressure valve (153) and to a pressure sensor (53) connected to the said processor (50), and is connected to at least one discharge solenoid valve (54) which is also controlled by the said processor (50);
- Any necessary means (56) for the temporary and removable connection of the processor (50) to any temperature probe (3,103) which may be housed in the base of the treatment container (1);
- Means (50) for activating the source of the microwaves (55) and for ensuring that, when the processor (50) detects the attainment of a predetermined temperature, by means of a temperature sensor (3) if present, or when it indirectly detects this value by means of the pressure sensor (53) which senses a corresponding value of the operating pressure, the said source (55) adapted to be kept active for a predetermined period which can be in accordance with the data acquired by the said temperature sensor and/or the pressure sensor, while the pressure within the container (1) is kept at around the said operating level by the modulated opening of the said solenoid valve (54), provision being made to ensure that, at the end of the cycle of sterilization of the waste placed in the treatment container, the said source of microwaves (55) is switched off, the said solenoid valve (54) is opened to discharge the residual pressure, and finally the said container (1) is removed from the piston (39) located above and is opened, provision being made to ensure that, during the active stage of operation of the station in question, if the pressure in the container (1) exceeds predetermined safety levels, the said safety valve (153) is triggered, and that, if the safety sensor (49) detects a pressure in excess of zero, the sterilization unit is automatically depressurized and opened, and an alarm signal is generated to warn of the occurrence of the problem, which is probably caused by the breaking of the seal (43) of the lower quartz piece (44) or by the breaking of this quartz piece.

12. Machine according to Claim 2, in which the station, if any, operating after the sterilization station and responsible for drying or dehydrating the sterilized waste in the treatment container (1) comprises a piston (58) with corresponding external seals (158) and comprises means (62) for inserting this piston into the mouth of the said container and vacuum means (61) are connected to the said piston and to its channels for internal connection to the container, for sucking from the latter the excess liquid contained in the sterilized waste.

13. Machine according to Claim 12, in which, in order to improve the operation of the said station for drying the treated waste, each treatment container (1) is provided in its base with at least one one-way valve which opens automatically to allow the passage of ambient air only when the said container is connected to the said vacuum source (61).

14. Machine according to Claim 1, in which the final station for discharging the sterilized waste from the treatment container (1) comprises:
- Means (63-69) for raising the container (1) with the waste from the corresponding lateral guides (9, 109) with the coupling of anti-rotation means (164, 264) with corresponding recessed parts (6, 106) provided in the shank (201) of the said container, these raising means being associated with and opposed to an annular hopper (73) which by reaction to the raising of the container (1) and in opposition to the action of elastic means (75) is inserted into the mouth of the container to protect it from contamination and to form an externally enlarged and diverging extension of the said mouth, the said raising means being provided with means (70, 71) for making the container (1) rotate into a substantially horizontal position during the final part of the said raising;
- Means, of a screw type for example (76-79), for extracting the treated waste from the container (1) in a substantially horizontal position, in such a way that the waste falls on to means of collection and evacuation;
- Means for returning the emptied container (1), between the corresponding vertical guides (9, 109) of the carousel, until it rests on the platform (107) of the said carousel, with the original angular orientation, while the assembly with the hopper (73) interacts with fixed means (268) which stop its downward travel in such a way that the said hopper is disengaged from the container (1) and is suitably removed from it, to allow the free transfer of the container by the carousel (7) to the next operating station.

15. Machine according to Claim 14, in which means are provided in the final operating station or after this station, to clean the treatment containers internally and if necessary to test the operation of the temperature probes, if present (3, 103).

16. Machine according to Claim 9, in which the sleeve (29) and the hopper (73), are adapted to be inserted into the mouth of the treatment container (1) during the loading and discharge of the waste into and from the said container, have such shapes and dimensions and are made from such materials that the whole of the upper inner part of the said container (1) designed to interact with the piston (39) of the waste sterilization station is kept clean.

17. Machine according to Claim 8, **characterized by** the use of a transfer, with horizontal axes for example, in place of the carousel with the vertical axis (7), for cyclically transferring the treatment containers (1) to the successive operating stations, means being provided to retain the said containers in the corresponding guides (9, 109) during the stage of running around the sprockets of the transfer and during travel along the lower branch of the said transfer, means of discharging the shredded waste and means, if any, of cleaning the said containers being made to operate during the stage in which the containers are positioned horizontally and orientated downwards.

18. Process for treating products with microwaves for sterilizing solid hospital waste stewed in suitable boxes (B) comprising the succession of the following operating steps:
- Fine shredding of the boxes with the waste and moistening with water, to precise levels, of the shredded product (V) obtained in this step;
- Insertion of a precise quantity of shredded and moistened waste into treatment containers (1) open at one end at least, and compaction of this waste, in such a way that the load of waste has characteristics of height, density and moisture content which are as far as possible constant and predetermined;
- Closing the said treatment container (1) to form a seal by means that connect the container by means of a wave guide (155) to a source (55) which generates microwaves at approximately 2.45 GHz for example and with suitable power, to raise the temperature of the waste and water within the said container to precise levels and to maintain this temperature for a time sufficient to ensure the sterilization of the said waste, while the internal pressure of the container is regulated by a controlled discharge towards the exterior, in such a way that predetermined maximum values are not exceeded, the treatment container with the sterilized waste being made to be depressurized and reopened at the end of the cycle;
- Discharge of the sterilized waste from the treatment containers, cleaning of the said containers (1) if necessary, and reintroduction of the containers into the operating cycle.

19. Process according to Claim 18, in which the sterilization step includes keeping the shredded and moistened waste at a temperature of approximately 150°C for a period of approximately 9-10 seconds at least, while the pressure within the treatment containers is kept below a specified maximum level, for example below 8 bars.

## Patentansprüche

1. Maschine zur Behandlung von Produkten mit Mikrowellen zur Sterilisierung von festen Krankenhausabfällen, die in geeigneten Pappkartons oder Wellkunststoffbehältern aufbewahrt sind, umfassend
eine Mehrzahl von robusten Behandlungsbehältern (1) beliebiger geeigneter Form und Kapazität, die an wenigstens einem Ende offen sind und angemessen druckfest und wärmebeständig sind und die durch die Bewegung eines Karussells (7) mit einer vertikalen Achse oder beliebiger geeigneter Fördermittel der Maschine mit wenigstens den nachfolgenden Betriebsstationen der Vorrichtung zyklisch in Wirkverbindung gebracht werden: eine erste Station, die zum Zerkleinern der Behälter mit den Abfällen ausgebildet ist, um ein fein gehäckseltes Produkt (V) zu erzeugen, das durch geeignete Mittel der Maschine in jeden Behälter (1), um einen vorbestimmten konstanten Wert kompaktiert und geeignet befeuchtet, gefüllt wird; eine zweite Station, die dazu ausgebildet ist, die Öffnung des Behälters dann, wenn sich die verdichtete, konstante und feuchte Abfallmasse darin befindet, durch geeignete Mittel (39) temporär abzudichten, die durch einen Wellenleiter (155) mit einer Quelle (55) in Verbindung stehen, die dazu ausgebildet ist, Mikrowellen mit der notwendigen Leistung und für eine notwendige Zeitspanne zu erzeugen, die feuchte Abfallmasse (V) zu sterilisieren, wobei diese Station zumindest mit Mitteln zur Überwachung und Steuerung des Drucks, der in dem Behälter während der Erwärmung mittels Mikrowellen erzeugt wird, und Mitteln zur Druckentlastung des Behälters am Ende des Zyklus ausgestattet ist, und eine letzte Station, die dazu ausgebildet ist, den sterilisierten Abfall aus dem Behälter auszugeben.

2. Maschine gemäß Anspruch 1, bei der zwischen der zweiten und der letzten Station eine dritte Station zum Entfeuchten oder Trocknen des sterilisierten Abfalls um ein ausreichendes Maß vorgesehen ist.

3. Maschine gemäß Anspruch 1, bei der Mittel zum Reinigen der Behandlungsbehälter (1) in der letzten Betriebsstation oder hinter dieser Station vorgesehen sind.

4. Maschine gemäß Anspruch 1, bei der Mittel zum Desinfizieren der Behandlungsbehälter (1) und, falls erforderlich, aller Bauteile der Maschine, die mit dem Abfall in Kontakt treten und sterilisiert werden sollen, bevor die Bauteile Wartungsarbeiten unterzogen werden, vorgesehen sind.

5. Maschine gemäß Anspruch 1, bei der die Behandlungsbehälter (1) mit einer äußeren Ummantelung zur Wärmeisolierung (2) versehen sind.

6. Maschine gemäß Anspruch 1, bei der jeder Behandlungsbehälter (1) wenigstens eine Temperatursonde (3) mit einem zugehörigen externen Ausgang (103) für eine entfernbare, schnelle und zeitweilige Verbindung mit Mitteln versehen ist, die ausgebildet sind, die Temperatur in dem Behälter während der Sterilisierung des Abfalls, der in diesem enthalten ist, zu bestimmen, wobei diese Sonde vorzugsweise so nah wie möglich zum Boden des Behälters angeordnet ist.

7. Maschine gemäß Anspruch 1, bei der jeder Behandlungsbehälter (1) aus einem Stahlzylinder geeigneter Größe und mit einem runden Querschnitt besteht, der eine geeignete innere trichterförmige Erweiterung (101) an seiner oberen Öffnung und eine geeignete Abrundung der eckigen Bereiche der Verbindung zwischen der Seitenfläche und dem Boden des Behälters aufweist.

8. Maschine gemäß Anspruch 1, bei der die Behandlungsbehälter (1) vertikal und mit ihren Öffnungen nach oben gerichtet an einem Karussell mit einer vertikalen Achse (7) gleitend zwischen vertikalen Führungen (9, 109), die an diesem Karussell befestigt sind, befestigt sind und wobei jeder der Behälter außen an seinen Boden mit einem Axialschaft (201) versehen ist, der einen Keil (4) aufweist, der durch die geformte Durchgangsbohrung (8, 108) in der Plattform (107) des Karussells hindurchtritt, unter der der Schaft (201) mit einer unteren Öffnung (5) in der Form des Buchstabens C, der um 90° gedreht ist, vorragt, und der durch die Anwesenheit des Keils auf einer theoretischen Ebene, die die Rotationsachse des Karussells enthält, derart gehalten wird, dass diese Öffnung durch den Kopf von gegenüberstehenden und/oder sich bewegenden Mitteln in Form eines Pilzes oder eines T, die in den Arbeitsstationen der Maschine angeordnet sind, korrekt in und außer Eingriff bringbar sind.

9. Maschine gemäß Anspruch 1, bei der die erste Station
- Mittel (10-18) zum Nacheinanderzuführen der Behälter mit Abfall (B) in den Einfülltrichter (16) einer Zerkleinerungsvorrichtung (23, 123), mit einer Pressvorrichtung (19), die ausgebildet ist, den Behälter zu den Mitteln zum Zerkleinern fortschreitend und in einer kontrollierten Weise vorzuschieben,
- Siebmittel (25), die nur erlauben, dass der Abfall, der ausreichend fein und auf eine vorbestimmte Partikelgröße zerkleinert ist, nach unten hindurchtreten kann,
- Fördermittel (26), die ausgebildet sind, dass der zerkleinerte Abfall, der die Siebmittel verlässt, gesammelt und zu einer Position oberhalb des Behälters (1), der gefüllt werden soll, transportiert wird,
- Mittel (27, 127), die so ausgebildet sind, dass überschüssige Flüssigkeit des zerkleinerten Abfalls gesammelt und abgeleitet wird,
- Mittel (33) zum korrekten Befeuchten des zerkleinerten Abfalls, der in den Behandlungsbehälter (1) eingefüllt werden soll und/ oder bereits eingefüllt ist,
- eine vertikale Hülse (29), deren unteres Ende durch beliebige geeignete Mittel um ein genaues Maß in die Öffnung des Behandlungsbehälters (1) zeitweilig eingeführt wird, der sich stationär in der Einfüllstation befindet und der mit einer seitlichen Öffnung (32) versehen ist, durch die der zerkleinerte Abfall, der von den Fördermittel (26) zugeführt wird, hindurchtritt,
- einen Kolben (34), der an einem vertikalen Schieber (230) befestigt ist, der beispielsweise durch einen Motor (35) mit einer elektronischen Geschwindigkeits-, Drehmoment- und Phasensteuerung angetrieben ist, und so ausgebildet ist, dass er einen Kolbenhub entlang der Führung zyklisch ausführt und den zerkleinerten Abfall, der sich in dem Behandlungsbehälter befindet, kompaktiert,
- Mittel zum zyklischen Laden einer Menge von zerkleinertem Abfall in den Behandlungsbehälter (1) mit einer Höhe und einer Dichte, die so weit wie möglich konstant und vorbestimmt sind, und Mittel zum Sicherstellen, dass diese Abfallmenge eine ausreichende Menge von Wasser enthält,
umfasst.

10. Maschine gemäß Anspruch 9, bei der Mittel (216) vorgesehen sind, die dazu geeignet sind, die gesamte Umgebung, in der der Abfall zerkleinert wird unter einem präzisen Unterdruck im Verhältnis zu der Außenumgebung zu halten und sicher und hygienisch jeglicher Staub, der beim Zerkleinern des Abfalls auftritt, zu entfernen und zurückzugewinnen.

11. Maschine gemäß Anspruch 1, bei der die Station zum Sterilisieren der Lademenge des zerkleinerten Abfalls, die sich in den Behandlungsbehältern (1) befindet, folgendes umfasst:
- Mittel (36, 37) zum zeitweiligen Anheben des Behälters (1) mit dem Abfall, der behandelt werden soll, derart, dass ein feststehender Kolben (39) in den Behälter mit einer seitlichen Dichtung und für einen genauen Weg eintritt, wobei der Kolben eine Axialkammer (42) aufweist, die durch den Wellenleiter (155) mit der Quelle (55), die ausgebildet ist, Mikrowellen mit der Frequenz und mit der Leistung, die für den Sterilisationsschritt notwendig ist, zu erzeugen, verbunden ist,
- in dem unteren Abschnitt der Axialkammer des Kolbens (39) wenigstens zwei Membranen (44, 44') aus einem mikrowellendurchlässigen, jedoch nicht flüssigkeitsdurchlässigen Werkstoff, beispielsweise Quarz, die in einem genauen Abstand zueinander vorgesehen sind, und wobei die Kammer, die zwischen diesen zwei Membranen ausgebildet ist, durch einen geeigneten Abstandshalter (45) begrenzt ist, der so ausgelegt ist, eine Verbindung mittels geeigneter Durchlässe (46-48) mit einem Drucksensor (49) herstellt, der mit dem Prozessor (50), der zur Steuerung des Systems ausgebildet ist, verbunden ist,
- einen an der unteren Fläche des Kolbens (39) offener Druckauslass (152), der durch einen Kanal (52) mit wenigstens zwei Abzweigungen zu einem Sicherheitsventil oder einem Druckbegrenzungsventil (153) und einem Drucksensor (53), der mit dem Prozessor (50) verbunden ist, verbunden ist und mit wenigstens einem Auslassmagnetventil (54) verbunden ist, das ebenfalls durch den Prozessor (50) gesteuert wird,
- beliebige notwendige Mittel (56) zur zeitweiligen und lösbaren Verbindung des Prozessors (50) mit einer Temperatursonde (3, 103), die in dem Boden des Behandlungsbehälters (1) aufgenommen sein kann,
- Mittel (50) zur Aktivierung der Quelle der Mikrowellen (55) und zum Sicherstellen, dass, wenn der Prozessor (50) das Erreichen einer vorbestimmten Temperatur mittels eines Temperatursensors (4), wenn dieser vorgesehen ist, erkennt oder wenn er indirekt diesen Wert mittels des Drucksensors (53) bestimmt, der einen entsprechenden Wert des Betriebsdrucks meldet, wobei die Quelle (55) ausgebildet ist, dass sie für einen vorbestimmten Zeitraum, der mit den Daten, die durch den Temperatursensor und/oder den Drucksensor festgestellt werden, aktiviert ist, halten, während der Druck in dem Behälter (1) ungefähr auf dem Betriebsniveau mittels der modulierten Öffnung des Magnetventils (54) gehalten wird, wobei sichergestellt wird, dass am Ende des Sterilisationszyklus des Abfalls, der sich in dem Behandlungsbehälter befindet, die Quelle der Mikrowellen (55) abgeschaltet wird, das Magnetventil (54) geöffnet wird, um den Restdruck entweichen zu lassen und schließlich der Behälter (1) von dem Kolben (39), der oberhalb angeordnet und geöffnet ist, entfernt wird, wobei sichergestellt wird, dass während des aktiven Betriebszustands dieser Station, dann, wenn der Druck in dem Behälter (1) über vorbestimmte Sicherheitsniveaus ansteigt, das Sicherheitsventil (153) ausgelöst wird, und dass dann, wenn der Sicherheitssensor (49) einen Druck größer Null misst, die Sterilisationseinheit automatisch druckentlastet und geöffnet und ein Alarmsignal erzeugt wird, um auf das Auftreten des Problems hinzuweisen, das vermutlich durch den Bruch der Dichtung (43) des unteren Quarzteils (44) oder durch den Bruch dieses Quarzteils hervorgerufen wurde.

12. Maschine gemäß Anspruch 2, bei der die Station, wenn sie vorhanden ist, und hinter der Sterilisationsstation betrieben wird und für das Trocknen und Entwässern des sterilisierten Abfalls in dem Behandlungsbehälter (1) verantwortlich ist, einen Kolben (58) mit zugehörigen externen Dichtungen (158) und Mittel (62) zum Einführen dieses Kolbens in die Öffnung des Behälters und Vakuummittel (61), die mit dem Kolben und seinen Kanälen für eine interne Verbindung mit dem Behälter zum Absaugen der übermäßigen Flüssigkeit, die in dem sterilisierten Abfall enthalten ist, verbunden sind, umfasst.

13. Maschine gemäß Anspruch 12, bei der, um den Betrieb der Station zum Trocknen des behandelten Abfalls zu verbessern, jeder Behandlungsbehälter (1) in seinem Boden mit wenigstens einem Einwegventil versehen ist, das automatisch öffnet, um den Einlass von Umgebungsluft nur dann zu ermöglichen, wenn der Behälter mit der Vakuumquelle (61) verbunden ist.

14. Maschine gemäß Anspruch 1, bei der die letzte Station zum Ausstoßen des sterilisierten Abfalls aus dem Behandlungsbehälter (1) folgendes umfasst:
- Mittel (63-69) zum Abheben des Behälters (1) mit dem Abfall von den entsprechenden seitlichen Führungen (9, 109) und den Antirotationskopplermitteln (164, 264) aus den entsprechenden Ausnehmungsabschnitten (6, 106), die im Schaft (201) des Behälters vorgesehen sind, wobei diese Abhebemittel mit einem ringförmigen Trichter (73) verbunden und diesem gegenüberliegend angeordnet sind, der als Folge des Abhebens des Behälters (1) und entgegen der Wirkung der elastischen Mittel (75) in die Öffnung des Behälters eingeführt wird, um diesen gegen Verunreinigungen zu schützen und um eine extern vergrößerte und auseinander strebende Verlängerung der Öffnung zu bilden, wobei die Abhebemittel mit Mitteln (70, 71) versehen sind, den Behälter (1) in einer im wesentlichen horizontalen Position während des letzten Abschnitts des Abhebens in Rotation zu versetzen,
- Mittel, beispielsweise eines Schraubentyps (76-79), zum Ausstoßen des behandelten Abfalls aus dem Behälter (1) in einer im wesentlichen horizontalen Position derart, dass der Abfall auf Mittel zum Sammeln und Entfernen fällt,
- Mittel zum Zurückstellen des leeren Behälters (1) zwischen die zugehörigen vertikalen Führungen (9, 109) des Karussells, bis er auf der Plattform (107) des Karussells mit der winkelmäßigen Ursprungsausrichtung ruht, wobei die Montageeinheit mit dem Trichter (73) mit ortsfesten Mitteln (268) zusammenwirkt, die seine Abwärtsbewegung derart unterbrechen, dass der Trichter von dem Behälter (1) gelöst wird und in einer geeigneten Weise davon entfernt wird, um den freien Transfer des Behälters mittels des Karussells (7) zu der nächsten Betriebsstation zu ermöglichen.

15. Maschine gemäß Anspruch 14, bei der in der letzten Betriebsstation oder hinter dieser Station Mittel vorgesehen sind, um die Behandlungsbehälter innen zu reinigen und wenn notwendig, die Funktion der Temperatursonden, wenn diese vorgesehen sind (3, 103), zu überprüfen.

16. Maschine gemäß Anspruch 9, bei der die Hülse (29) und der Trichter (73), die so ausgebildet sind, dass sie in die Öffnung des Behandlungsbehälters (1) während des Beladens des Abfalls in und der Ausgabe aus dem Behälter einführbar sind und solch eine Form und Dimensionen aufweisen und aus solchen Werkstoffen hergestellt sind, dass der gesamte obere innere Abschnitt des Behälters (1), der dazu ausgebildet ist, mit dem Kolben (39) der Abfallsterilisationsstation zusammenzuwirken, sauber gehalten wird.

17. Maschine gemäß Anspruch 8, **gekennzeichnet durch** die Verwendung einer Transfereinheit, beispielsweise mit horizontalen Achsen, anstelle des Karussells mit der vertikalen Achse (7), zur zyklischen Weiterleitung der Behandlungsbehälter (1) zu den nachfolgenden Betriebsstationen, wobei Mittel vorgesehen sind, um die Behälter in den entsprechenden Führungen (9, 109) während des Umlaufs um die Kettenräder der Transfereinheit und während des Transportwegs entlang des unteren Zweigs der Transfereinheit zu halten, **durch** Mittel zum Ausstoßen des zerkleinerten Abfalls und Mittel, falls vorgesehen, die dann zum Reinigen der Behälter während des Stadiums ausgelegt sind, in dem die Behälter horizontal positioniert und nach unten gerichtet sind.

18. Verfahren zum Behandeln von Produkten mit Mikrowellen zum Sterilisieren von festen Krankenhausabfällen, die in geeigneten Behältern (B) aufbewahrt werden, umfassend die Folge folgender Arbeitsschritte
- Feines Zerkleinern der Behälter mit Abfall und Befeuchten mit Wasser auf genaue Niveaus des in diesem Schritt erzielten Produkts (V),
- Einführen einer genauen Menge von zerkleinertem und befeuchtetem Abfall in Behandlungsbehälter (1), die wenigstens an einem Ende offen sind, und Kompaktieren dieses Abfalls derart, dass die Abfallmenge eine Höhe, eine Dichte und einen Feuchtigkeitsgehalt aufweist, welche so weit wie möglich konstant und vorbestimmt sind,
- Verschließen der Behandlungsbehälter (1) um eine Dichtung durch Mittel zu bilden, die den Behälter mittels eines Wellenleiters (155) mit einer Quelle (55) verbinden, die beispielsweise Mikrowellen von ungefähr 2,45 GHz und mit einer geeigneten Leistung erzeugt, um die Temperatur des Abfalls und des Wassers in dem Behälter auf genaue Niveaus anzuheben und diese Temperatur für einen Zeitraum, der ausreicht, um die Sterilisation des Abfalls sicherzustellen, aufrecht zu erhalten, während der Innendruck des Behälters durch ein geregeltes Entlasten zur Außenumgebung derart, dass vorbestimmte maximale Werte nicht überschritten werden, wobei der Behandlungsbehälter mit dem sterilisierten Abfall am Ende des Zyklus druckentlastet und wieder geöffnet wird,
- Ausstoßen des sterilisierten Abfalls aus den Behandlungsbehältern, Reinigen der Behälter (1), falls notwendig, und Wiedereinführen der Behälter in den Arbeitszyklus.

19. Verfahren gemäß Anspruch 18, bei dem der Sterilisationsschritt das Halten des zerkleinerten und feuchten Abfalls auf einer Temperatur von ungefähr 150°C für wenigstens einen Zeitraum von ungefähr 9 - 10 Sekunden umfasst, während der Druck in dem Behandlungsbehälter unterhalb eines vorgegebenen maximalen Werts, beispielsweise unterhalb von 8 bar, gehalten wird.

## Revendications

1. Machine permettant de traiter des produits à l'aide de micro-ondes afin de stériliser des déchets hospitaliers solides qui sont déposés dans des boîtes appropriées en plastique ondulé ou en carton, comprenant une pluralité de conteneurs de traitement robustes (1) présentant une forme et une capacité quelconques, ouverts à une extrémité au moins et résistants à la chaleur et à la pression d'une manière appropriée qui, sous l'action d'un carrousel ayant un axe vertical ou d'un autre moyen de transport approprié quelconque de la machine conçu pour être élaboré de façon cyclique pour interagir avec au moins les postes de commande suivants de la machine : un premier poste conçu pour déchiqueter les boîtes contenant des déchets, pour créer un produit finement déchiqueté (V) qui, par un moyen approprié de la machine, est introduit et compacté en quantités constantes prédéterminées, humecté de manière appropriée dans chaque conteneur (1) ; un deuxième poste conçu pour sceller provisoirement l'ouverture du conteneur, avec la masse de déchets humectée, compactée et constante à l'intérieur, par un moyen approprié (39) qui communique au moyen d'un guide d'ondes (155) avec une source (55) conçue pour générer les micro ondes avec la puissance nécessaire et pendant la durée nécessaire pour stériliser ladite masse de déchets humectée (V), ce poste étant équipé au moins de moyens permettant de surveiller et de commander la pression générée dans le conteneur au cours du chauffage par micro-ondes et de moyens permettant de dépressuriser le conteneur à la fin du cycle : et un poste final conçu pour refouler les déchets stérilisés du conteneur.

2. Machine selon la revendication 1, dans laquelle un troisième poste de déshydratation ou de séchage des déchets stérilisés jusqu'à un degré suffisant est prévu entre le deuxième poste et le poste final.

3. Machine selon la revendication 1, dans laquelle des moyens permettant de nettoyer les conteneurs de traitement (1) sont prévus au niveau du poste de commande final ou après ce poste.

4. Machine selon la revendication 1, dans laquelle des moyens sont prévus pour désinfecter le conteneur de traitement (1) et si nécessaire toutes les pièces de la machine qui sont en contact avec les déchets stérilisés avant que lesdites pièces soient soumises à des opérations de maintenance.

5. Machine selon la revendication 1, dans laquelle les conteneurs de traitement (1) sont dotés d'une enveloppe externe assurant une isolation thermique (2).

6. Machine selon la revendication 1, dans laquelle chaque conteneur de traitement (1) porte au moins une sonde de température (3) ayant une sortie externe correspondante (103) permettant une connexion provisoire, rapide et amovible au moyen conçu pour détecter la température à l'intérieur dudit conteneur au cours de l'étape de stérilisation des déchets contenus à l'intérieur, cette sonde étant de préférence placée le plus près possible de la base dudit conteneur

7. Machine selon la revendication 1, dans laquelle chaque conteneur de traitement (1) est composé d'un cylindre en acier de dimensions appropriées ayant une coupe ronde et un évasement interne (101) approprié sur son ouverture supérieure et un arrondi approprié de la région angulaire de la connexion entre la surface latérale et la base dudit conteneur.

8. Machine selon la revendication 1, dans laquelle les conteneurs de traitement (1) sont montés verticalement, leurs ouvertures étant placées vers le haut sur un carrousel ayant un axe vertical (7), avec la possibilité de coulisser entre les guides verticaux (9, 109) fixés sur ce carrousel, et chacun desdits conteneurs est doté, à l'extérieur, sur sa base, d'une tige axiale (201) qui possède une clavette (4) qui traverse le trou traversant conformé (8, 108) sur la plate-forme (107) du carrousel, en dessous duquel lesdites tiges (201) font saillie avec une ouverture inférieure (5) en forme de C tournée de quatre-vingt dix degrés, et qui est maintenue grâce à la présence de ladite clavette sur un plan théorique contenant l'axe de rotation du carrousel, de telle sorte que cette ouverture puisse être engagée et dégagée correctement par la tête d'un moyen mobile et/ou opposé présentant la forme d'un champignon ou d'un T, situé au niveau des postes de commande de la machine

9. Machine selon la revendication 1, dans laquelle le premier poste de commande comprend :
- des moyens (10-18) permettant d'introduire les déchets (B) dans les boîtes, un à la fois dans la trémie d'alimentation (16) d'un broyeur (23, 123), avec un presseur (19) conçu pour pousser progressivement et d'une manière commandée ladite boîte vers le moyen de broyage ;
- un moyen de criblage (25) qui ne permet qu'aux déchets broyés de manière à présenter une taille de particules prédéterminée suffisamment fine, de passer vers le bas;
- un moyen de transport (26) conçu pour récupérer les déchets broyés quittant ledit moyen de criblage et de les amener jusqu'à une position située au-dessus du conteneur (1) à remplir ;
- des moyens (27, 127) conçus pour récupérer et drainer l'excès de liquide provenant des déchets broyés ;
- un moyen (33) permettant d'humecter correctement les déchets broyés à insérer ou déjà insérés dans le conteneur de traitement (1) ;
- un manchon vertical (29) dont l'extrémité inférieure est insérée provisoirement par un moyen approprié quelconque sur une distance précise dans l'entrée du conteneur de traitement (1) qui est fixe au niveau du poste de chargement, et qui est doté d'une ouverture latérale (32) à travers laquelle passent les déchets broyés arrivant dudit moyen de transport (26) ;
- un piston (34) monté sur un coulisseau vertical (230) entraîné, par exemple par un moteur (35) doté d'une commande de phase, couple et vitesse électronique conçu pour déplacer d'une manière cyclique ledit piston le long dudit guide et comprimer les déchets broyés placés dans le conteneur de traitement ;
- un moyen permettant de charger cycliquement une quantité de déchets broyés dans le conteneur de traitement (1), avec des valeurs de hauteur et de densité qui sont le plus possible constantes et prédéterminées, et un moyen permettant de garantir que cette charge de déchets contient une quantité suffisante d'eau.

10. Machine selon la revendication 9, dans laquelle un moyen approprié (216) est prévu pour maintenir tout l'environnement dans lequel sont broyés les déchets à une pression négative précise par rapport à l'environnement externe, et retirer et récupérer d'une manière sûre et hygiénique toute poussière résultant de ladite étape de broyage des déchets

11. Machine Selon la revendication 1, dans laquelle le poste de stérilisation de la charge de déchets broyés placés dans le conteneur de traitement (1) comprend :
- des moyens (36, 37) permettant d'élever provisoirement le conteneur (1) contenant les déchets à traiter de telle sorte qu'un piston fixe (39) pénètre dans ledit conteneur avec une étanchéité latérale et sur une distance précise, le piston ayant une cavité axiale (42) qui est connectée par un guide d'ondes (155) à la source (55) conçue pour produire les micro-ondes à la fréquence et avec la puissance requises pour l'étape de stérilisation ;
- dans la partie inférieure de ladite cavité axiale du piston (39), au moins deux diaphragmes (44, 44') composés d'un matériau non perméable aux fluides mais perméable aux micro-ondes, comme du quartz, sont prévus à une distance précise l'un de l'autre, et la chambre formée entre ces deux diaphragmes, délimitée par une entretoise appropriée (45), est conçue pour assurer une connexion, par le biais de passages appropriés (46, 48), à un capteur de pression (49) connecté au processeur (50) conçu pour commander le fonctionnement du système ;
- sur la face inférieure dudit piston (39) s'ouvre une sortie de pression (152) qui est connectée, au moyen d'un canal (52) ayant au moins deux ramifications, à une soupape de sûreté ou une soupape de pression maximale (153) et à un capteur de pression (53) connecté audit processeur (50), et est connectée à au moins une électrovalve de décharge (54) qui est également commandée par ledit processeur (50) ;
- des moyens quelconques nécessaires (56) permettant la connexion temporaire et amovible du processeur (50) à une sonde de température (3, 103) quelconque qui peut être logée dans la base du conteneur de traitement (1),
- un moyen (50) permettant d'activer la source de micro-ondes (55) et de garantir que lorsque le processeur (50) détecte qu'une température prédéterminée a été atteinte, au moyen d'un capteur de température (3), s'il existe, ou lorsqu'il détecte indirectement cette valeur au moyen du capteur de pression (53) qui détecte une valeur correspondante de la pression opérationnelle, ladite source (55) étant conçue pour être maintenue active pendant une période prédéterminée qui peut être fonction des données acquises par ledit capteur de température et/ou le capteur de pression, tandis que la pression à l'intérieur du conteneur (1) est maintenue à peu près audit niveau opérationnel grâce à l'ouverture modulée de ladite électrovalve (54), pour garantir que, à la fin du cycle de stérilisation des déchets placés dans le conteneur de traitement, ladite source de micro-ondes (55) est arrêtée, ladite électrovalve (54) est ouverte pour refouler la pression résiduelle et enfin ledit conteneur (1 ) est retiré du piston (39) situé au-dessus et ouvert, pour garantir qu'au cours de l'étape active de fonctionnement du poste en question, si la pression du conteneur (1) dépasse des niveaux de sécurité prédéterminés, ladite soupape de sûreté (153) est déclenchée, et que, si le capteur de sûreté (49) détecte une pression supérieure à zéro, l'unité de stérilisation est automatiquement dépressurisée et ouverte, et un signal d'alarme est généré pour avertir du problème, qui est probablement provoqué par la rupture du joint (43) de l'élément à quartz inférieur (44) ou par la rupture de l'élément en q ua rtz.

12. Machine selon la revendication 2, dans laquelle le poste s'il existe, fonctionnant après le poste de stérilisation et garantissant le séchage et la déshydratation des déchets stérilisés dans le conteneur de traitement (1) comprend un piston (58) ayant des joints externes correspondants (158) et comprend un moyen (62) permettant d'insérer ce piston dans l'ouverture dudit conteneur et un moyen de vide (61) est connecté audit piston et à ses canaux pour assurer une connexion interne au conteneur, pour aspirer dans celui-ci l'excès de liquide contenu dans les déchets stérilisés.

13. Machine selon la revendication 12, dans laquelle, afin d'améliorer le fonctionnement dudit poste permettant de sécher les déchets traités, chaque conteneur de traitement (1) est doté dans sa base d'au moins un clapet unidirectionnel qui s'ouvre automatiquement pour permettre le passage de l'air ambiant uniquement lorsque ledit conteneur est connecté à ladite source de vide (61).

14. Machine selon la revendication 1, dans laquelle le poste final permettant de refouler les déchets stérilisés du conteneur de traitement (1) comprend :
- des moyens (63-69) permettant d'élever le conteneur (1) contenant les déchets à partir des guides latéraux correspondants (9, 109), le couplage des moyens anti-rotation (164-264) avec les parties évidées correspondantes (6, 106) étant prévu dans la tige (201) dudit conteneur ces moyens d'élévation étant associés et opposés à une trémie annulaire (73) qui en réaction à l'élévation du conteneur (1) et en opposition à l'action du moyen élastique (75), est insérée dans l'entrée du conteneur pour la protéger de toute contamination et former une extension divergente et agrandie de l'extérieur de ladite entrée, ledit moyen d'élévation étant doté de moyens (70, 71) pour faire tourner le conteneur (1) dans une position sensiblement horizontale au cours de la partie finale de ladite élévation ;
- des moyens, de type vis par exemple, (76-79) permettant d'extraire les déchets traités du conteneur (1) dans une position sensiblement horizontale, de telle sorte que les déchets débouchent sur des moyens de récupération et d'évacuation ;
- des moyens permettant de renvoyer le conteneur vide (1), entre les guides verticaux correspondants (9, 109) du carrousel, jusqu'à ce qu'il repose sur la plate-forme (107) dudit carrousel, avec l'orientation angulaire originale, tandis que l'ensemble comprenant les trémies (73) interagit avec les moyens fixes (268) qui arrêtent son déplacement vers le bas de telle sorte que ladite trémie est dégagée du conteneur (1) et retirée de manière appropriée de celui-ci, pour permettre un transfert libre du conteneur par le carrousel (7) jusqu'au poste de commande suivant.

15. Machine selon la revendication 14, dans laquelle des moyens sont prévus au niveau du poste de commande final ou après ce poste pour nettoyer l'intérieur des conteneurs de traitement et si nécessaire tester le fonctionnement des sondes de température, si elles existent (3, 103).

16. Machine selon la revendication 9, dans laquelle le manchon (29) et la trémie (73) qui sont conçus pour être insérés dans l'ouverture du conteneur de traitement (1) au cours du chargement et du refoulement des déchets dans ledit et dudit conteneur présentent des formes et des dimensions et sont composés de matériaux, permettant que la totalité de la partie interne supérieure dudit conteneur (1) conçue pour interagir avec le piston (39) du poste de stérilisation des déchets soit maintenue propre.

17. Machine selon la revendication 8, **caractérisée par** l'utilisation d'un transfert, ayant des axes horizontaux par exemple, à la place du carrousel ayant les axes verticaux (7), pour transférer de manière cyclique les conteneurs de traitement (1) vers les postes de commande successifs, un moyen étant prévu pour retenir lesdits conteneurs dans les guides correspondants (9, 109) au cours de l'étape de déplacement autour des roues dentées du transfert et au cours du déplacement le long de la ramification inférieure dudit transfert, un moyen de refoulement des déchets broyés et un moyen, s'il existe, de nettoyage desdits conteneurs étant mis en fonctionnement au cours de l'étape pendant laquelle les conteneurs sont placés horizontalement et orientés vers le bas.

18. Procédé permettant de traiter des produits à l'aide de micro-ondes pour stériliser des déchets hospitaliers solides broyés dans des boîtes appropriées (B) comprenant la succession des étapes d'exploitation suivantes :
- broyage fin des boîtes contenant les déchets et humidification avec de l'eau jusqu'à des niveaux précis du produit broyé (V) obtenu au cours de cette étape ;
- insertion d'une quantité précise de déchets humectés et broyés dans des conteneurs de traitement (1) ouverts à une extrémité au moins et compactage de ces déchets de telle sorte que la charge de déchets présente des caractéristiques de hauteur, densité et humidité le plus possible constantes et prédéterminées ;
- fermeture dudit conteneur de traitement (1) pour former un joint grâce à un moyen qui connecte le conteneur, à l'aide d'un guide d'ondes (155) à une source (55) qui génère des micro-ondes à environ 2,45 GHz par exemple et avec une puissance appropriée, pour élever la température des déchets et de l'eau contenus dans ledit conteneur jusqu'à des niveaux précis et maintenir cette température pendant une durée suffisante pour garantir la stérilisation desdits déchets, tandis que la pression interne du conteneur est régulée par un refoulement contrôlé vers l'extérieur, de telle sorte que les valeurs maximums prédéterminées ne soient pas dépassées, le conteneur de traitement contenant les déchets stérilisés étant élaboré pour être dépressurisé et rouvert à la fin du cycle ;
- refoulement des déchets stérilisés des conteneurs de traitement, nettoyage desdits conteneurs (1) si nécessaire et réinsertion des conteneurs dans le cycle d'exploitation.

19. Procédé selon la revendication 18, dans lequel l'étape de stérilisation comporte le maintien des déchets humectés et broyés à une température d'environ 150 °C pendant une période d'environ 9 à 10 secondes au moins, tandis que la pression dans les conteneurs de traitement est maintenue à une valeur inférieure à un niveau maximum spécifié, par exemple inférieure à 8 bars.
